# EUROPÄISCHE PATENTSCHRIFT

(11) **EP 2 934 616 B1**
(45) Veröffentlichungstag und Bekanntmachung des Hinweises auf die Patenterteilung: **31.01.2018**
(21) Anmeldenummer: 13811343.6
(22) Anmeldetag: 12.12.2013
(51) Int. Cl.: A61M 1/06

(54) **BRUSTHAUBENEINHEIT**
BREAST SHIELD UNIT
UNITÉ DE TÉTERELLE

(30) Priorität: 18.12.2012 CH 28402012
(43) Veröffentlichungstag der Anmeldung: 28.10.2015
(73) Patentinhaber: Medela Holding AG, 6340 Baar (CH)
(72) Erfinder: FURRER, Etienne, CH-6332 Hagendorn (CH); RIGERT, Mario, CH-6033 Buchrain (CH); SCHLIENGER, André, CH-8933 Maschwanden (CH)
(74) Vertreter: Clerc, Natalia
(86) Internationale Anmeldenummer: PCT/CH2013/000219
(87) Internationale Veröffentlichungsnummer: WO 2014/094186

(56) Entgegenhaltungen:
- EP-A1- 1 500 408
- EP-A1- 2 308 523
- WO-A1-2010/054174
- WO-A1-2011/007140
- DE-A1- 19 816 776
- DE-U1-202006 019 416
- US-A- 3 822 703
- US-A1- 2003 004 459
- US-A1- 2007 060 873
- US-A1- 2011 251 552

## Beschreibung

### TECHNISCHES GEBIET

Die vorliegende Erfindung betrifft eine Brusthaubeneinheit gemäss Oberbegriff des Patentanspruchs 1.

### STAND DER TECHNIK

Brustpumpen zum Abpumpen von menschlicher Muttermilch mittels Unterdruck sind hinlänglich bekannt. Auf dem Markt sind manuell betriebene wie auch motorisch betriebene Brustpumpen erhältlich. Sie sind entweder direkt oder über eine Vakuumleitung mit einer Brusthaube verbunden. Die Brusthaube wird an die abzupumpende Mutterbrust angelegt, so dass mindestens die Brustwarze, meistens auch die Areola und das umliegende Gewebe der Mutterbrust, dichtend umschlossen sind. Die Brusthaube ist üblicherweise entweder direkt oder über eine Milchleitung mit einem Milchsammelbehälter verbunden, so dass abgepumpte Milch direkt in diesen Behälter fliessen kann.

Die bekannten Brusthauben sind im Wesentlichen starre Trichter, so dass je nach Grösse der Brust ein anderer Trichter gewählt werden muss. Ferner werden diese Brusthauben oft mit flexiblen und weichen Brusthaubeneinsätzen verwendet, um den Komfort für die Mutter zu erhöhen. Einige dieser Einsätze üben eine Massagewirkung auf die Brust aus, um so den Milchfluss zu fördern.

US 5 571 084 offenbart eine Brusthaubeneinheit mit einer halbkugelförmigen steifen Schale und einem darin angeordneten flexiblen Brusthaubeneinsatz. Der Brusthaubeneinsatz weist einen trichterförmigen Grundkörper und einen daran angeformten rohrförmigen Stutzen auf. Der Grundkörper ist mit einem umlaufenden Kragen über einen Flansch der Schale gestülpt. Der Stutzen ist in einer rohrförmigen Aufnahme der Schale fixiert gehalten.

WO 2011/007140 zeigt eine Brusthaubeneinheit mit einer steifen äusseren Brusthaube und einem flexiblen Brusthaubeneinsatz. Auch dieser Einsatz ist trichterförmig mit einem angeformten rohrförmigen Stutzen und er ist an beiden Enden an der Brusthaube befestigt.

WO 2011/037841 beschreibt einen Brusthaubeneinsatz, an welchem ein Entenschnabelventil angeordnet ist.

EP 2 308 523, DE 198 16 776, DE 20 2006019416U, US 2007/060873 und US 3822 703 offenbaren Brusthauben in der klassischen Trichterform mit teilweise verschiebbaren Einsätzen.

### DARSTELLUNG DER ERFINDUNG

Es ist eine Aufgabe der Erfindung, eine Brusthaubeneinheit und einen Brusthaubeneinsatz zu schaffen, welche optimal an die jeweilige Form der Mutterbrust anpassbar sind und diese möglichst gleichmässig belastet bzw. beansprucht.

Diese Aufgabe löst eine Brusthaubeneinheit mit den Merkmalen gemäss Anspruch 1.

Die erfindungsgemässe Brusthaubeneinheit zur Verwendung mit einer Brustpumpe zum Abpumpen von menschlicher Muttermilch weist eine formstabile Brusthaube und einen flexiblen Brusthaubeneinsatz zur Einlage in die Brusthaube auf. Die Brusthaube weist ein erstes offenes Brusthaubenende zur Anlage an eine Mutterbrust auf. Der Brusthaubeneinsatz weist ein erstes und ein zweites Einsatzende auf, welche eine Längsrichtung definieren, wobei das erste offene Brusthaubenende einen umlaufenden Rand aufweist, an welchem der Brusthaubeneinsatz mit dem ersten Einsatzende befestigbar oder befestigt ist. Der Brusthaubeneinsatz erstreckt sich im montierten Zustand von diesem ersten Einsatzende im Innern der Brusthaube zum zweiten Einsatzende. Der Brusthaubeneinsatz verläuft zwischen dem ersten und dem zweiten Einsatzende im Wesentlichen beabstandet zur Brusthaube. Erfindungsgemäss ist das zweite Einsatzende im montierten Zustand des Brusthaubeneinsatzes in Längsrichtung relativ zur Brusthaube verschiebbar.

Diese Brusthaubeneinheit schmiegt sich dank des in Längsrichtung verschiebbaren Endes des Brusthaubeneinsatzes optimal an die Mutterbrust an. Diese Anpassung lässt sich durch Flexibilität und Dünnwandigkeit des Brusthaubeneinsatzes noch verbessern. Üblicherweise drücken Mütter aus Angst, dass die Brusthaube nicht dicht genug auf der Brust anliegt, die Brusthaube zu stark an die Brust an. Dies ist nicht nur unbequem und bei längerem Gebrauch schmerzhaft, sondern beeinträchtigt auch den Milchfluss. Ein derart übermässiges Andrücken wird durch die erfindungsgemässe Brusthaubeneinheit verhindert. Die Anlage an die Brust und auch die Auflagekraftverteilung ist optimiert.

Ein weiterer Vorteil ist, dass dank der Anpassungsfähigkeit des Brusthaubeneinsatzes dieselbe Form und Grösse der Brusthaubeneinheit und des Brusthaubeneinsatzes für die Verwendung innerhalb eines grossen Bereichs unterschiedlicher Formen und Grössen von Brüsten geeignet ist. Dies erleichtert der Mutter die Wahl der Brusthaubeneinheit und reduziert die Herstellungskosten.

Diese Anpassungsfähigkeit an die Mutterbrust lässt sich durch geeignete Wahl einer dem ersten Einsatzende des Brusthaubeneinsatzes benachbarten Brustauflage, welche eine Auflagefläche zur Auflage auf die Mutterbrust aufweist, erhöhen. Zum Beispiel kann diese Brustauflage gedehnt oder vorgespannt sein. Zusätzlich oder alternativ kann diese Brustauflage annähernd senkrecht zur Längsrichtung verlaufen.

Der erfindungsgemässe Brusthaubeneinsatz weist vorzugsweise einen rohrförmigen Stutzen auf, welcher das zweite Einsatzende bildet. Dieser Einsatz lässt sich kostengünstig herstellen. Dank seiner einfachen Form ist er einfach zu reinigen.

Der erfindungsgemässe Brusthaubeneinsatz weist in einer bevorzugten Ausführungsform ein erstes Einsatzende und ein dem ersten Einsatzende gegenüberliegendes zweites Einsatzende auf, welche gemeinsam eine Längsrichtung definieren. Benachbart zum ersten Einsatzende ist ein Auflagebereich zur Auflage auf die Mutterbrust ausgebildet. Ein rohrförmiger Stutzen ist zur Aufnahme des Nippels der Mutterbrust vorhanden. Er ist an dem Auflagebereich angeformt, und er erstreckt sich bis zum zweiten Einsatzende. Erfindungsgemäss ist die Auflagefläche des Auflagebereichs annähernd eben ausgebildet.

Die Länge des Brusthaubeneinsatzes in der genannten Längsrichtung beträgt dabei ein Vielfaches der Länge des Auflagebereichs in dieser Längsrichtung.

Dieser Brusthaubeneinsatz ist optimal eingesetzt, wenn sein zweites freies Ende gegenüber der Brusthaube verschiebbar gehalten ist. Er lässt sich jedoch auch mit beiden Enden einspannen bzw. befestigen. Die annähernd ebene Auflagefläche, insbesondere wenn im montierten Zustand vorgespannt, ermöglicht ebenfalls eine optimierte Anpassung an unterschiedliche Brustgrössen und verhindert durch seine Form ein zu starkes Anpressen. Dies wird hier als weitere Erfindung ausgewiesen.

Das zweite hintere Einsatzende kann auch mit einem Gehäuse unverschiebbar verbunden sein, welches verschiebbar in der Brusthaube gehalten ist. Ist ein Gehäuse vorhanden, so lässt sich dieses einfach manuell verschieben und hilft beim Anlegen an die Mutterbrust.

In einer bevorzugten Ausführungsform ist ferner eine Medientrenneinrichtung vorhanden, welche ein extern angelegtes Vakuum in das Innere des Brusthaubeneinsatzes überträgt, gleichzeitig jedoch verhindert, dass abgepumpte Milch zur Vakuumquelle oder in allfällige Vakuumleitungen gelangen kann. Vorzugsweise ist in diesem Fall das zweite Einsatzende des Brusthaubeneinsatzes mit der Medientrenneinrichtung verbunden, welches das oben genannte Gehäuse bildet.

Vorzugsweise ist die Medientrenneinrichtung als Ganzes relativ zur Brusthaube in Längsrichtung bewegbar angeordnet. Auch dies ist eine eigenständige Erfindung, welche auch ohne Brusthaubeneinsatz bzw. ohne Brusthaubeneinsatz mit verschiebbarem zweitem Ende in anderen Brusthauben zur Anwendung gelangen kann.

Vorzugsweise ist die Medientrennvorrichtung mittels der flexiblen Brustauflage am Gehäuse aufgehängt. In einer bevorzugten Ausführungsform erfolgt diese Aufhängung dadurch, dass ein umlaufender Befestigungskragen der Brustauflage über einen Flansch der Brusthaube gestülpt ist.

In einer bevorzugten Ausführungsform weist die Brusthaube sich in Längsrichtung erstreckende Führungsschlitze auf, entlang welcher die Medientrenneinrichtung als Ganzes bewegbar ist. Dadurch ist die Bewegung geführt und begrenzt.

Vorzugsweise weist die Medientrenneinrichtung ein formstabiles Gehäuse und eine darin bewegbar angeordnete Medientrennmembran auf.

In einer bevorzugten Ausführungsform weist die Brusthaube an ihrem dem ersten Ende abgewandten zweiten Ende eine rückseitige Öffnung auf, wodurch das zweite Ende des Brusthaubeneinsatzes und somit der gesamte Brusthaubeneinsatz durch Zugang über diese rückseitige Öffnung in Längsrichtung zur Brust hin manuell verschiebbar ist. Dies erleichtert das Aufsetzen und Anpassen der Brusthaubeneinheit an die Mutterbrust. Die Brustauflage des Brusthaubeneinsatzes lässt sich nach vorne aus der Brusthaube heraus drücken. Je nach Form lässt sie sich sogar umstülpen. Wird nun diese Brustauflage an die Brust angelegt, so nimmt sie automatisch die Form der Brust an. Je nach Form klappt sie dabei in diese angepasste Form um.

Ist eine Medientrenneinrichtung vorhanden und ist das zweite Ende des Brusthaubeneinsatzes mit dieser Medientrenneinrichtung verbunden, so ist vorzugsweise die Medientrenneinrichtung durch Zugang über das offene zweite Ende der Brusthaube manuell eindrückbar bzw. verschiebbar.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: einen Längsschnitt durch eine erfindungsgemässe Brusthaubeneinheit, montiert auf einem Milchsammelbehälter, in einer ersten Ausführungsform;
- Figur 2: die Brusthaubeneinheit mit Milchsammelbehälter gemäss Figur 1 bei Auflage auf eine Mutterbrust;
- Figur 3: einen Längsschnitt durch einen erfindungsgemässen Brusthaubeneinsatz;
- Figur 4: eine perspektivische Darstellung des Brusthaubeneinsatzes gemäss Figur 3 von vorne;
- Figur 5: eine perspektivische Darstellung des Brusthaubeneinsatzes gemäss Figur 3 von hinten;
- Figur 6: einen Längsschnitt durch eine erfindungsgemässe Brusthaubeneinheit, montiert auf einem Milchsammelbehälter, in einer zweiten Ausführungsform;
- Figur 7: die Brusthaubeneinheit gemäss Figur 6 in einer vergrösserten Darstellung;
- Figur 8: eine perspektivische Darstellung der Brusthaubeneinheit gemäss Figur 6 in einer ersten Position;
- Figur 9: die Brusthaubeneinheit gemäss Figur 7 beim Anlegen auf eine Mutterbrust;
- Figur 10: eine perspektivische Darstellung der Brusthaubeneinheit in der Position gemäss Figur 9;
- Figur 11: die Brusthaubeneinheit gemäss Figur 7 bei Auflage auf die Mutterbrust und
- Figur 12: die Brusthaubeneinheit gemäss Figur 7 in einer geneigten Position.

### BESCHREIBUNG BEVORZUGTER AUSFÜHRUNGSFORMEN

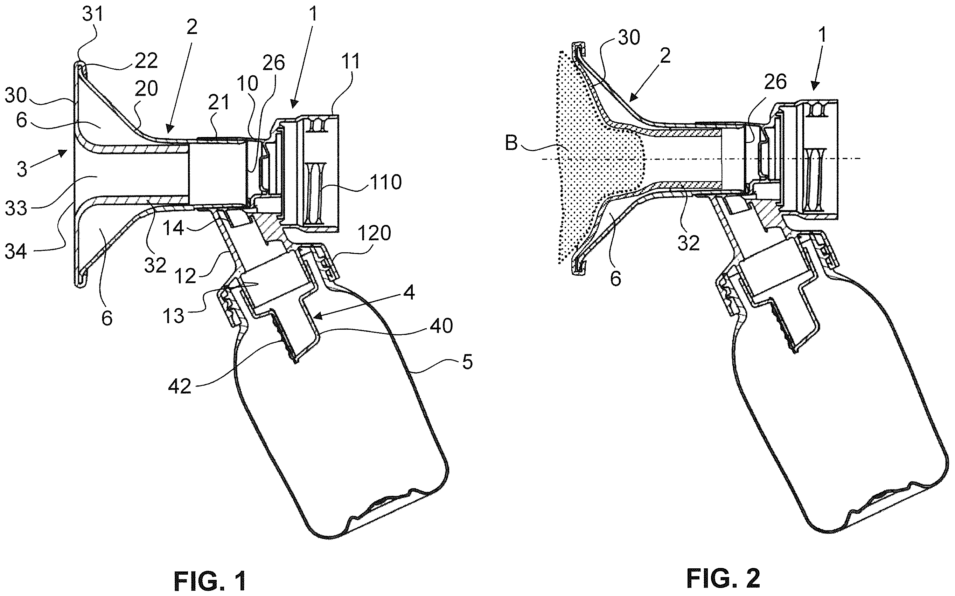

In den Figuren 1 und 2 ist ein erstes Beispiel einer erfindungsgemässen Brusthaubeneinheit dargestellt. Sie weist eine Brusthaube 2 und einen Brusthaubeneinsatz 3 auf.

Die Brusthaube 2 weist einen Trichterkörper 20 zur Aufnahme einer Mutterbrust B und einen daran vorzugsweise einteilig angeformten rohrförmigen Stutzen 21 auf. Das dem Stutzen 21 abgewandte brustseitige Ende der Brusthaube 2 ist mit einem umlaufenden, nach aussen kragenden Flansch 22 versehen. Die Brusthaube 2 ist vorzugsweise steif oder zumindest formstabil ausgebildet. Sie ist vorzugsweise aus Kunststoff gefertigt.

Die Brusthaube 2 ist in einem Kopplungsteil 1 gehalten. Das Kopplungsteil 1 weist einen vorzugsweise steifen Grundkörper 10 auf mit einer rohrförmigen Aufnahme für den Stutzen 21 der Brusthaube 2. Ein Vakuumanschlussteil 11 dient dem Anschluss einer manuell oder motorisch betriebenen Vakuumpumpe. Alternativ oder zusätzlich dient das Anschlussteil 11 zum Einstecken einer Vakuumleitung, welche eine Verbindung zu einer externen Vakuumpumpe schafft. Das Vakuumanschlussteil 11 kann ein Innengewinde 110 aufweisen oder anderweitig ausgebildet sein.

Abgewinkelt zu diesem Vakuumanschlussteil 11 ist ein Behälteranschlussteil 12 mit einer Milchauslassöffnung 14 vorhanden, welcher zur Verbindung mit einem Milchsammelbehälter 5 dient. Auch dieses Anschlussteil 12 weist einen Anschlussstutzen 120 oder ein anderweitig geeignetes Anschlussmittel auf. Im Innern des Kopplungsteils 1, hier im Hohlraum des Anschlussstutzens 120, ist vorzugsweise ein Ventil angeordnet, um das Totvolumen beim Abpumpen zu begrenzen und ein Rückfliessen der abgepumpten Milch zu verhindern. In diesem Beispiel ist hierzu ein Hals 13 am Kopplungsteil 1 angeformt, auf welchem eine Ventileinheit 4 mit einem Ventilkörper 40 und einer Ventilmembran 42 befestigbar ist.

Das Kopplungsteil 1 und der Milchsammelbehälter 5 können auch eine andere Form aufweisen. Das Kopplungsteil 1 kann auch Bestandteil der Brusthaube 2 sein, wie beispielsweise im weiter unten beschriebenen Beispiel gezeigt ist. Dabei kann eine einteilige oder mehrteilige Einheit aus Kopplungsteil 1 und Brusthaube 2 gebildet sein.

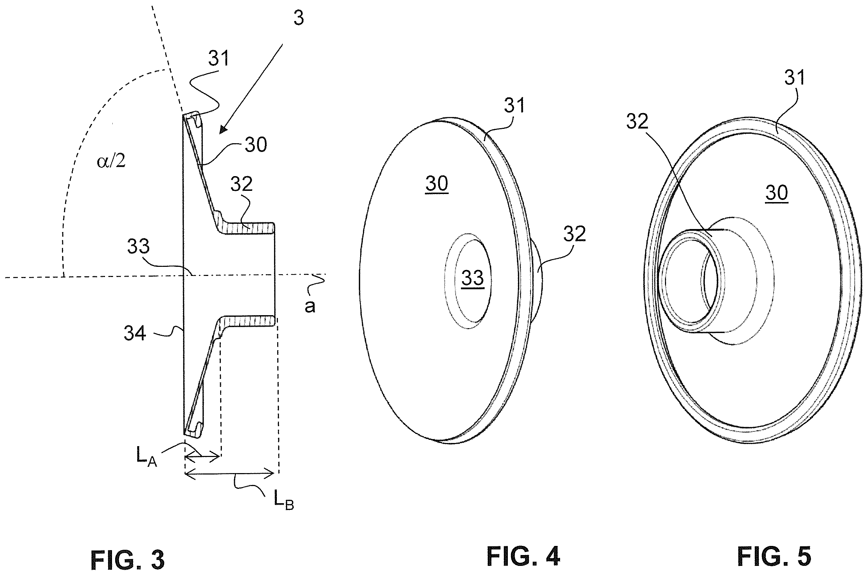

Der Brusthaubeneinsatz 3 ist aus einem weichen, elastischen und flexiblen Material gefertigt, beispielsweise aus Silikon. Er weist eine Brustauflage 30 mit einer Aufnahmeöffnung 33 zur Auflage auf die Mutterbrust B und einen daran einteilig angeformten Stutzen 32 auf. Die Aufnahmeöffnung 33 führt vom brustnahen Ende des Einsatzes 3 in einem Kanal durch den Stutzen 32 bis zum brustfernen Ende des Einsatzes 3. Dies definiert eine Längsrichtung a, welche in Figur 3 angegeben ist.

An einem brustseitigen, dem Stutzen 32 abgewandten ersten Einsatzende 34 des Einsatzes 3 ist ein Befestigungskragen 31 angeformt, welcher um den Flansch 22 der Brusthaube 2 gestülpt werden kann. Dadurch lässt sich der Brusthaubeneinsatz 3 an der Brusthaube 2 befestigen.

Der Stutzen 32 des Brusthaubeneinsatzes 3 ist in den Stutzen 21 der Brusthaube 2 eingeschoben. Er ist jedoch in seinem Aussendurchmesser so ausgestaltet, dass er innerhalb dieses Stutzens 21 gleitet und somit nicht fixiert ist. Der Stutzen 32 des Brusthaubeneinsatzes 3 bildet somit ein zweites Einsatzende, welches relativ zur Brusthaube 2 verschiebbar gehalten ist.

In Figur 1 ist die Brusthaubeneinheit im montierten, aber unbenützten oder nicht aktuell benützten Zustand dargestellt. In diesem Zustand ist zwischen Brusthaube 2 und Brusthaubeneinsatz 3 ein umlaufender Hohlraum 6 vorhanden, d.h. der Brusthaubeneinsatz 3, oder zumindest die Brustauflage 30 und der nicht eingesteckte Teil des Stutzens 32, verläuft beabstandet zur Innenwand der Brusthaube 2. Die Brustauflage 30 ist annähernd eben oder sie weist eine Trichterform mit einem relativ grossen Öffnungswinkel α von 100 bis 150° auf. Vorzugsweise ist die Brustauflage 30 gedehnt oder vorgespannt in der Brusthaube 2 gehalten. D.h. sie weist im nicht montierten Zustand vorzugsweise einen Öffnungswinkel α von 120° bis 180° auf und ist im montierten Zustand annähernd eben oder bis zum oben genannten Öffnungswinkel gedehnt oder vorgespannt.

In Figur 2 ist die Brusthaubeneinheit im benützten Zustand dargestellt. Die Brustauflage 30 ist auf eine Mutterbrust B aufgelegt, wobei die Brustwarze in die Aufnahmeöffnung 33 hineinragt. Durch Anpressen der Brusthaubeneinheit auf die Brust B verformt sich einerseits die Brustauflage 30 und passt sich an die Form der Brust B an. Sie nimmt insbesondere, aber nicht ausschliesslich, eine Trichterform und/oder eine konkave Form an. Bei zu starkem Druck verschiebt sich der Brusthaubeneinsatz 3 nach hinten, d.h. von der Brust B weg, indem sich der Stutzen 32 relativ zur Brusthaube 2, und somit zur vorderen Befestigung des Einsatzes 3 in der Brusthaube 2, verschiebt und sich einem hinteren Ende 26 der Brusthaube 2 nähert. Das dichte Anliegen des Einsatzes 3 auf der Brust B ist dabei nach wie vor gewährleistet, auch wenn der Anpressdruck vermindert ist.

In den Figuren 3 bis 5 ist ein derartiger Brusthaubeneinsatz 3 dargestellt. Er entspricht im Wesentlichen demjenigen gemäss den Figuren 1 und 2, wobei sein Stutzen 32 kürzer ausgebildet ist. Zudem weist die Brustauflage 30 eine kleinere Wandstärke auf als der Stutzen 32. Der Stutzen 32 ist vorzugsweise annähernd formstabil ausgebildet und die Brustauflage 30 membranähnlich und somit flexibler und elastischer als der Stutzen 32. Die Brustauflage 30 und die Aufnahmeöffnung 33 sind vorzugsweise kreisförmig ausgebildet. Vorzugsweise ist die Brustauflage 30 vollständig flach ausgebildet, wobei auch ihre Rückseite, d.h. die der Brust abgewandte Seite, vorzugsweise keine Erhebungen und Vertiefungen aufweist. Die Brustauflage 30 weist eine Länge L_{A} auf, welche um ein Vielfaches kleiner ist als die Länge L_{B} des gesamten Einsatzes 3.

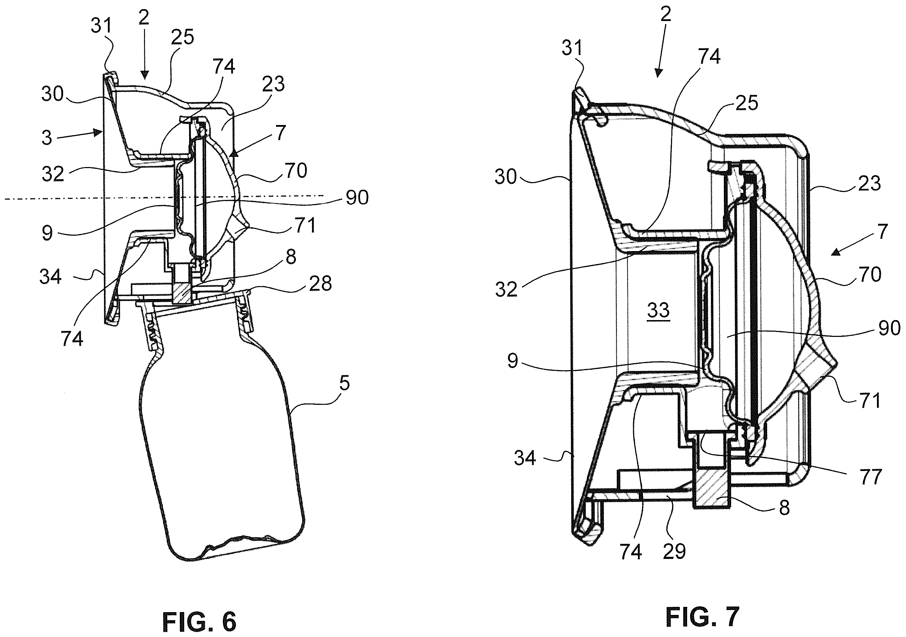

In den Figuren 6 bis 12 ist ein weiteres Ausführungsbeispiel dargestellt. Gleiche Teile sind mit gleichen Bezugszeichen wie im ersten Beispiel versehen.

In dieser Ausführungsform weist die Brusthaube 2 einen gehäuseförmigen Grundkörper 25 auf. Die Brusthaube 2 dient zur Auflage auf die Mutterbrust B, aber auch gleichzeitig zur Verbindung mit dem Milchsammelbehälter 5. Sie weist hierfür ein Milchanschlussteil 28 auf, mit welchem sie mit dem Milchsammelbehälter 5 verbunden werden kann. Die Brusthaube 2 ist vorzugsweise steif oder formstabil und insbesondere aus Kunststoff gefertigt.

Im Grundkörper 25 ist eine Medientrenneinrichtung 7 angeordnet, welcher hier zur Medientrennung und zur Verbindung mit dem Brusthaubeneinsatz 3 und der nicht dargestellten Vakuumpumpe dient. Medientrenneinrichtungen für Brustpumpen sind aus dem Stand der Technik bekannt. Sie dienen dazu, die Vakuumpumpe vor Verschmutzung durch Milch zu schützen.

Die Medientrenneinrichtung 7 gemäss den Figuren 6 bis 12 weist ein Gehäuse 70 auf, welches vorzugsweise steif oder formstabil und insbesondere aus Kunststoff ausgebildet ist. Im hinteren, der Brust B abgewandten Bereich ist ein Vakuumanschluss 71 zur Verbindung mit einer Vakuumpumpe oder einer Vakuumleitung vorhanden.

Eine Milchauslassöffnung 77, welche mit einem Rückschlagventil 8 versehen ist, führt durch eine Durchführungsöffnung 29 des Grundkörpers 25 in den Milchsammelbehälter 5.

Am Gehäuse 70 der Medientrenneinrichtung 7 ist eine Brusthaubeneinsatz-Aufnahme 74 angeformt, welche einen Aufnahmestutzen für den Stutzen 32 des Einsatzes 3 bildet. Der Stutzen 32 des Einsatzes 3 ist bei Gebrauch unverschieblich in dieser Aufnahme 74 gehalten. Er ist beispielsweise in einem Presssitz gehalten und kann zwecks Reinigung oder Ersatz entfernt werden. Er kann jedoch auch nicht zerstörungsfrei lösbar mit der Medientrenneinrichtung 7 verbunden sein. Das erste Einsatzende 34 des Einsatzes 3 ist wiederum über den Flansch 22 des Grundkörpers 25 der Brusthaube 2 gestülpt. Der Einsatz 3 ist vorzugsweise gemäss dem in den Figuren 3 bis 5 dargestellten Ausführungsbeispiel bzw. im oben beschriebenen erfindungsgemässen Sinne ausgebildet. Vorzugsweise weist der Einsatz 3 in allen Ausführungsbeispielen einen maximalen Aussendurchmesser auf, welcher leicht kleiner ist als der maximale Aussendurchmesser der Brusthaube 2. Vorzugsweise weist der Befestigungskragen 31 einen kleineren Durchmesser auf als der Flansch 22 der Brusthaube 2. Dadurch wird der Einsatz 3 beim Befestigen auf der Brusthaube 2 gedehnt.

Die Medientrenneinrichtung 7 weist zwecks Medientrennung eine Medientrennmembran 9 auf. Diese ist im Gehäuse 70 bewegbar gehalten. Sie bildet mit der Rückwand des Gehäuses 70 eine Pumpkammer 90. Wird über den Vakuumanschluss 71 zyklisch ein Unterdruck in die Pumpkammer geleitet, so bewegt sich die Medientrennmembran 9 synchron dazu und überträgt den Unterdruck in den Hohlraum, gebildet durch den Brusthaubeneinsatz 3 und die Mutterbrust B. Milch wird abgepumpt. Die Medientrennmembran 9 verhindert dabei, dass abgepumpte Milch in die Pumpkammer 90 und somit zur Vakuumquelle gelangen kann. Die Milch fliesst vielmehr durch die der Membran 9 vorgelagerte Milchauslassöffnung 77 in den Milchsammelbehälter 5.

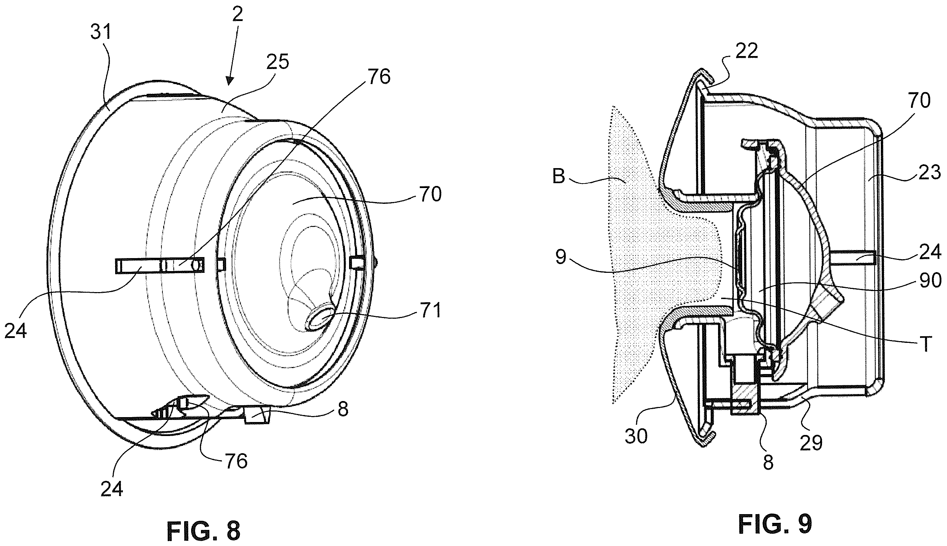

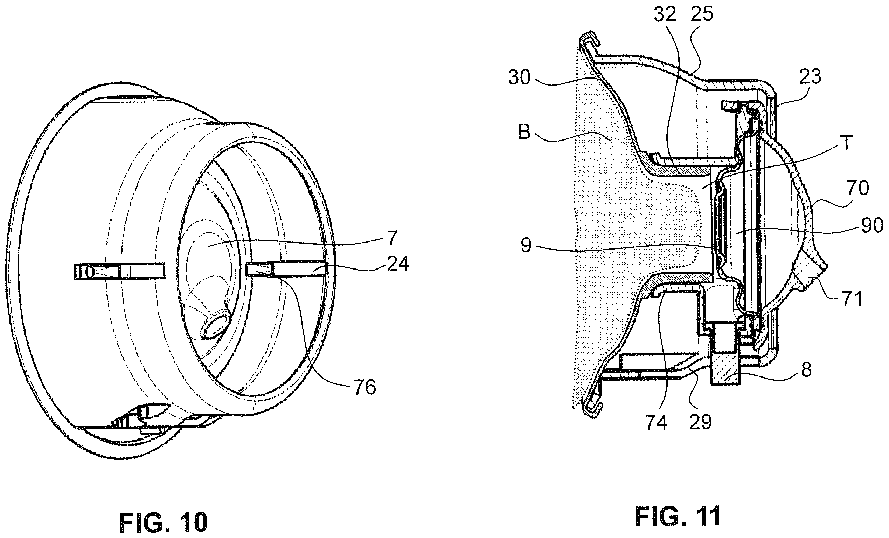

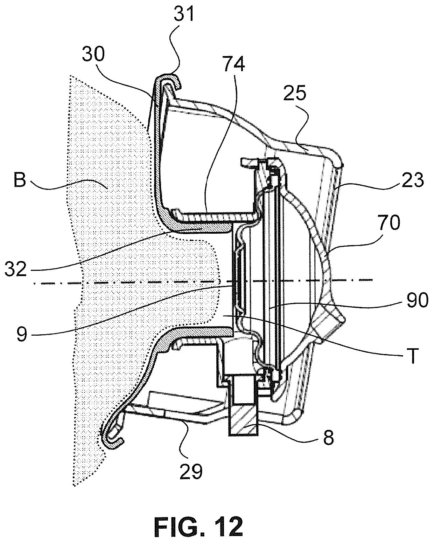

Vorzugsweise befindet sich die Medientrennmembran 9 mindestens im nicht ausgelenkten Ausgangszustand im Auflagebereich der Brustwarze. Dies minimiert das Totvolumen T optimal. Wie in Figur 9 erkennbar ist, reicht die Medientrennmembran 9 bereits im nicht ausgelenkten Zustand annähernd oder genau bis zum Ende des Stutzens 32 des Einsatzes 3. In Figur 11 ist erkennbar, dass der Stutzen 32 so bemessen ist, dass er bei einer im Normgrössenbereich liegenden Brust und Brustwarze in seiner gesamten Länge von der Brustwarze ausgefüllt ist. Die Medientrennmembran 9 berührt jedoch während des Abpumpens die Brustwarze vorzugsweise nicht, sondern sie bewegen sich synchron in die gleiche Richtung. In Figur 12 ist eine geneigte Position dargestellt, welche durch entsprechend geformte Führungsschlitze 24 erhalten werden kann.

Die Medientrenneinrichtung 7 ist erfindungsgemäss beweglich, insbesondere verschiebbar im Grundkörper 25 der Brusthaube 2 angeordnet. Hierzu weist der Grundkörper 25 Führungsschlitze 24 auf, in welche Führungsnasen 76 des Gehäuses 70 eingreifen. Dies ist beispielsweise in Figur 8 erkennbar. Die Medientrenneinrichtung 7 lässt sich entlang dieser Schlitze 24 verschieben. Die Schlitze 24 können geradlinig oder gekrümmt verlaufen. In diesem Beispiel sind sie geradlinig. Das Ventil 8 und die Milchauslassöffnung 77 führen in allen Positionen der Medientrenneinrichtung 7 relativ zum Grundkörper 25 in den Milchsammelbehälter 5, da die Durchführöffnung 29 grösser ausgebildet ist als das Ventil 8 und die Milchauslassöffnung 77. Vorzugsweise ist die Durchführungsöffnung 29 als Langloch ausgebildet.

Das hintere, der Brust abgewandte Ende des Grundkörpers 25 ist offen ausgebildet. Die Medientrenneinrichtung 7 lässt sich manuell über diese rückseitige Öffnung 23 bewegen, hier entlang der Schlitze 24 verschieben.

In den Figuren 7 und 8 ist die Brusthaubeneinheit im unbenützten bzw. aktuell nicht benützten Zustand dargestellt. Die Medientrenneinrichtung 7 ragt durch die rückseitige Öffnung 23 heraus oder befindet sich zumindest in einem hinteren Bereich. Die Brustauflage 30 des Einsatzes 3 ist entspannt und in diesem Beispiel leicht trichterförmig.

Um die Brusthaubeneinheit zu verwenden, wird die Medientrenneinrichtung 7 von Hand nach vorne gestossen, wie dies in den Figuren 9 und 10 dargestellt ist. Die Brustauflage 30 des Einsatzes 3 wird vorgespannt, je nach Bedarf nach vorne zur Mutterbrust B hin gestülpt. Die Brustwarze kann optimal in der Aufnahmeöffnung 33 aufgenommen werden. Wird nun der manuelle Druck auf das Gehäuse 70 gelöst, der Grundkörper 25 jedoch nach wie vor von Hand, in einem Büstenhalter oder einer anderen Haltevorrichtung an die Brust gehalten, so schiebt sich das Gehäuse 70 entlang der Schlitze 24 nach hinten und der Einsatz 3 liegt dichtend, jedoch ohne übermässigen bzw. ungleichmässig verteilten Druck an der Brust B an. Dies ist in Figur 11 dargestellt. Das Abpumpen kann beginnen.

Auch in diesem Beispiel ist somit das zweite Einsatzende relativ zur Brusthaube 2 verschiebbar, wobei die Verschiebung gemeinsam mit der Medientrenneinrichtung 7 erfolgt. Anstelle der Medientrenneinrichtung lässt sich das zweite Einsatzende auch in einem anderen relativ zur Brusthaube verschiebbaren Teil halten. Z.B. kann das Gehäuse 70 gleich wie oben beschrieben oder ähnlich ausgebildet und in der Brusthaube 2 gehalten sein, jedoch keine Medientrennmembran 9 aufweisen.

Die erfindungsgemässe Brusthaubeneinheit passt sich optimal der Form der Mutterbrust an und optimiert die Auflagekraft.

**BEZUGSZEICHENLISTE**

| | | | |
|---|---|---|---|
| 1 | Kopplungsteil | 4 | Ventileinheit |
| 10 | Grundkörper | 40 | Ventilkörper |
| 11 | Vakuumanschlussteil | 42 | Ventilmembran |
| 110 | Innengewinde | | |
| 12 | Behälteranschlussteil | 5 | Milchsammelbehälter |
| 120 | Anschlussstutzen | | |
| 13 | Hals | 6 | Hohlraum |
| 14 | Milchauslassöffnung | | |
| | | 7 | Medientrenneinrichtung |
| | | 70 | Gehäuse |
| 2 | Brusthaube | 71 | Vakuumanschluss |
| 20 | Trichterkörper | 74 | Brusthaubeneinsatz- |
| 21 | Stutzen | | Aufnahme |
| 22 | Flansch | 76 | Führungsnase |
| 23 | rückseitige Öffnung | 77 | Milchauslassöffnung |
| 24 | Führungsschlitz | | |
| 25 | Grundkörper | 8 | Ventil |
| 26 | Ende | | |
| 28 | Milchanschlussteil | 9 | Medientrennmembran |
| 29 | Durchführungsöffnung | 90 | Pumpkammer |
| | | | |
| 3 | Brusthaubeneinsatz | B | Brust |
| 30 | Brustauflage | T | Totvolumen |
| 31 | Befestigungskragen | a | Längsrichtung |
| 32 | Stutzen | α | Öffnungswinkel |
| 33 | Aufnahmeöffnung | L_{B} | Länge des Brusthaubeneinsatzes |
| 34 | erstes Einsatzende | | |
| | | L_{A} | Länge des Auflagebereichs |

## Patentansprüche

1. Brusthaubeneinheit zur Verwendung mit einer Brustpumpe zum Abpumpen von menschlicher Muttermilch mittels Unterdruck, wobei die Brusthaubeneinheit eine formstabile Brusthaube (2) und einen flexiblen Brusthaubeneinsatz (3) zur Einlage in die Brusthaube (2) aufweist, wobei die Brusthaube (2) ein erstes offenes Brusthaubenende zur Anlage an eine Mutterbrust (B) aufweist, wobei der Brusthaubeneinsatz (3) ein erstes und ein zweites Einsatzende (34, 32), eine dem ersten Einsatzende (34) benachbarte Brustauflage (30) sowie einen Stutzen (32) aufweist, wobei die Brustauflage (30) eine Auflagefläche zur Auflage auf die Mutterbrust (B) und eine Aufnahmeöffnung (33) aufweist, wobei der Stutzen (32) einteilig an der Brustauflage (30) angeformt ist, wobei das erste und das zweite Einsatzende (34, 32) eine Längsrichtung (a) definieren, wobei das erste offene Brusthaubenende einen umlaufenden Rand (22) aufweist, an welchem der Brusthaubeneinsatz (3) mit dem ersten Einsatzende (34) befestigbar oder befestigt ist, wobei sich der Brusthaubeneinsatz (3) im montierten Zustand von diesem ersten Einsatzende (34) im Innern der Brusthaube (2) zum zweiten Einsatzende (32) erstreckt und wobei der Brusthaubeneinsatz (3) zwischen dem ersten und dem zweiten Einsatzende (34, 32) im Wesentlichen beabstandet zur Brusthaube (2) verläuft, wobei das zweite Einsatzende (32) im montierten Zustand des Brusthaubeneinsatzes (3) in Längsrichtung (a) relativ zur Brusthaube (2) verschiebbar ist, **dadurch gekennzeichnet, dass** die Brustauflage (30) vor Auflage auf die Mutterbrust (B) annähernd senkrecht zur Längsrichtung (a) verläuft und vor Auflage auf die Mutterbrust (B) annähernd eben ausgebildet ist.

2. Brusthaubeneinheit nach Anspruch 1, wobei die Brustauflage (30) gedehnt oder vorgespannt ist.

3. Brusthaubeneinheit nach einem der Ansprüche 1 bis 2, wobei die Brusthaube (2) an ihrem dem ersten Ende abgewandten zweiten Ende eine rückseitige Öffnung (23) aufweist, wodurch das zweite Einsatzende (32) durch Zugang über diese rückseitige Öffnung (23) in Längsrichtung (a) zur Mutterbrust (B) hin manuell verschiebbar ist.

4. Brusthaubeneinheit nach einem der Ansprüche 1 bis 3, wobei das zweite Einsatzende (32) mit einer Medientrenneinrichtung (7, 9) verbunden ist, welche ein extern angelegtes Vakuum in das Innere des Brusthaubeneinsatzes (3) überträgt.

5. Brusthaubeneinheit nach Anspruch 4, wobei die Medientrenneinrichtung (7,9) als Ganzes relativ zur Brusthaube (2) in Längsrichtung (a) bewegbar angeordnet ist.

6. Brusthaubeneinheit nach Anspruch 5, wobei die Brusthaube (2) sich in Längsrichtung (a) erstreckende Führungsschlitze (24) aufweist, entlang welcher die Medientrenneinrichtung (7, 9) als Ganzes bewegbar ist.

7. Brusthaubeneinheit nach einem der Ansprüche 4 bis 6, wobei die Medientrenneinrichtung (7, 9) ein formstabiles Gehäuse (70) und eine darin bewegbar angeordnete Medientrennmembran (9) aufweist.

8. Brusthaubeneinheit nach einem der Ansprüche 3 bis 6, wobei die Brusthaube (2) an ihrem dem ersten Ende abgewandten zweiten Ende eine rückseitige Öffnung (23) aufweist, wodurch die Medientrenneinrichtung (7, 9) durch Zugang über diese rückseitige Öffnung (23) in Längsrichtung (a) zur Brust hin manuell verschiebbar ist.

9. Brusthaubeneinheit nach einem der Ansprüche 1 bis 8, wobei der Brusthaubeneinsatz (3) einen rohrförmigen Stutzen (32) aufweist, welcher das zweite Einsatzende bildet.

10. Brusthaubeneinheit nach einem der Ansprüche 1 bis 9, wobei (3) die Länge (L_{B}) des Brusthaubeneinsatzes (3) in der genannten Längsrichtung ein Vielfaches der Länge (L_{A}) des Auflagebereichs (30) in dieser Längsrichtung (a) beträgt.

11. Brusthaubeneinheit gemäss den Ansprüchen 7 und 10, wobei das zweite Einsatzende (32) unverschiebbar im Gehäuse (70) gehalten ist.

## Claims

1. Breastshield unit for use with a breastpump for expressing human breastmilk by means of underpressure, wherein the breastshield unit has a dimensionally stable breastshield (2) and a flexible breastshield insert (3) for placing in the breastshield (2), wherein the breastshield (2) has a first, open breastshield end for placing on a mother's breast (B), wherein the breastshield insert (3) has a first insert end (34) and a second insert end (32), a breast pad (30) adjoining the first insert end (34) as well as a stub (32), wherein the breast pad (30) comprises a bearing surface for bearing on the mother's breast (B) and a receiving opening (33), wherein the stub (32) is formed integrally on the breast pad (30), wherein the first and second insert ends (34, 32) define a longitudinal direction (a), wherein the first, open breastshield end has a peripheral edge (22), on which the breastshield insert (3) is or can be secured with the first insert end (34), wherein the breastshield insert (3), in the assembled state, extends from this first insert end (34) in the inside of the breastshield (2) to the second insert end (32), and wherein the breastshield insert (3), between the first and second insert ends (34, 32), extends substantially spaced apart from the breastshield (2), wherein, in the assembled state of the breastshield insert (3), the second insert end (32) is displaceable in the longitudinal direction (a) relative to the breastshield (2), **characterized in that** the breast pad (30) extends approximately perpendicularly with respect to the longitudinal direction (a) prior to the bearing on the mother's breast (B) and is designed approximately flat prior to the bearing on the mother's breast (B).

2. Breastshield unit according to Claim 1, wherein the breast pad (30) is stretched or pretensioned.

3. Breastshield unit according to one of Claims 1 to 2, wherein the breastshield (2) has a rear opening (23) at its second end directed away from the first end, as a result of which the second insert end (32) is manually displaceable in the longitudinal direction (a) towards the mother's breast (B) by access through this rear opening (23).

4. Breastshield unit according to one of Claims 1 to 3, wherein the second insert end (32) is connected to a media separation device (7, 9), which transfers an externally applied vacuum into the interior of the breastshield insert (3).

5. Breastshield unit according to Claim 4, wherein the media separation device (7, 9) as a whole is arranged to be movable relative to the breastshield (2) in the longitudinal direction (a).

6. Breastshield unit according to Claim 5, wherein the breastshield (2) has guide slits (24) which extend in the longitudinal direction (a) and along which the media separation device (7, 9) as a whole is movable.

7. Breastshield unit according to one of Claims 4 to 6, wherein the media separation device (7, 9) has a dimensionally stable housing (70) and a media separation membrane (9) arranged movably therein.

8. Breastshield unit according to one of Claims 3 to 6, wherein the breastshield (2) has a rear opening (23) at its second end directed away from the first end, as a result of which the media separation device (7, 9) is manually displaceable towards the breast in the longitudinal direction (a) by access through this rear opening (23).

9. Breastshield unit according to one of Claims 1 to 8, wherein the breastshield insert (3) has a tubular stub (32), which forms the second insert end.

10. Breastshield unit according to one of Claims 1 to 9, wherein the length (L_{B}) of the breastshield insert (3) in said longitudinal direction is a multiple of the length (L_{A}) of the bearing area (30) in this longitudinal direction (a).

11. Breastshield unit according to one of Claims 7 to 10, wherein the second insert end (32) is held non-displaceably in the housing (70).

## Revendications

1. Unité de téterelle destinée à être utilisée avec un tire-lait pour le pompage de lait maternel humain par dépression, l'unité de téterelle présentant une téterelle (2) indéformable et un insert de téterelle (3) flexible destiné à être introduit dans la téterelle (2), la téterelle (2) présentant une première extrémité de téterelle ouverte destinée à être posée contre un sein maternel (B), l'insert de téterelle (3) présentant une première et une deuxième extrémité d'insert (34, 32), un appuiesein (30) voisin de la première extrémité d'insert (34) ainsi qu'un embout (32), l'appuie-sein (30) présentant une surface d'appui destinée à venir en appui sur le sein maternel (B) et une ouverture de réception (33), l'embout (32) étant formé d'une seule partie sur l'appuie-sein (30), la première et la deuxième extrémité d'insert (34, 32) définissant une direction longitudinale (a), la première extrémité de téterelle ouverte présentant un bord périphérique (22) sur lequel l'insert de téterelle (3) avec la première extrémité d'insert (34) peut être fixé ou est fixé, l'insert de téterelle (3) s'étendant, dans l'état monté, à partir de cette première extrémité d'insert (34) à l'intérieur de la téterelle (2) vers la deuxième extrémité d'insert (32), et l'insert de téterelle (3) se situant entre la première et la deuxième extrémité d'insert (34, 32) essentiellement à distance de la téterelle (2), la deuxième extrémité d'insert (32) pouvant, dans l'état monté de l'insert de téterelle (3), être déplacée dans la direction longitudinale (a) par rapport à la téterelle (2), **caractérisée en ce que**, avant la pose sur le sein maternel (B), l'appuie-sein (30) est placé approximativement perpendiculairement à la direction longitudinale (a) avant la pose sur le sein maternel (B) et est constitué de façon approximativement plane avant la pose sur le sein maternel (B).

2. Unité de téterelle selon la revendication 1, l'appuie-sein (30) étant étiré où pré-tendu.

3. Unité de téterelle selon l'une des revendications 1 à 2, la téterelle (2) présentant une ouverture (23) côté arrière à sa deuxième extrémité éloignée de la première extrémité, ce qui fait que la deuxième extrémité d'insert (32) peut être déplacée manuellement dans la direction longitudinale (a) vers le sein maternel (B) par l'accès via cette ouverture (23) côté arrière.

4. Unité de téterelle selon l'une des revendications 1 à 3, la deuxième extrémité d'insert (32) étant raccordée à un dispositif de séparation de milieux (7, 9) qui transmet un vide appliqué de façon externe vers l'intérieur de l'insert de téterelle (3).

5. Unité de téterelle selon la revendication 4, le dispositif de séparation de milieux (7, 9) étant disposé dans son ensemble de façon mobile dans la direction longitudinale (a) par rapport à la téterelle (2).

6. Unité de téterelle selon la revendication 5, la téterelle (2) présentant des fentes de guidage (24) s'étendant dans la direction longitudinale (a), le long desquelles le dispositif de séparation de milieux (7, 9) est mobile dans son ensemble.

7. Unité de téterelle selon l'une des revendications 4 à 6, le dispositif de séparation de milieux (7, 9) présentant un boîtier (70) indéformable et une membrane de séparation de milieux (9) qui y est disposée de façon mobile.

8. Unité de téterelle selon l'une des revendications 3 à 6, la téterelle (2) présentant une ouverture (23) côté arrière à sa deuxième extrémité éloignée de la première extrémité, ce qui fait que le dispositif de séparation de milieux (7, 9) peut être déplacé manuellement dans la direction longitudinale (a) vers le sein par l'accès via cette ouverture (23) côté arrière.

9. Unité de téterelle selon l'une des revendications 1 à 8, l'insert de téterelle (3) présentant un embout (32) de forme tubulaire qui forme la deuxième extrémité d'insert.

10. Unité de téterelle selon l'une des revendications 1 à 9, (3) la longueur (L_{B}) de l'insert de téterelle (3) étant, dans la direction longitudinale citée, égale à un multiple de la longueur (L_{A}) de la zone d'appui (30) dans cette direction longitudinale (a).

11. Unité de téterelle selon les revendications 7 et 10, la deuxième extrémité d'insert (32) étant retenue de façon non déplaçable dans le boîtier (70).
